# EUROPEAN PATENT APPLICATION

(11) **EP 3 513 736 A1**
(43) Date of publication of application: **24.07.2019**
(21) Application number: 17850993.1
(22) Date of filing: 14.09.2017
(51) Int. Cl.: A61B 8/13

(54) **PHOTOACOUSTIC IMAGE GENERATION APPARATUS**

(30) Priority: 14.09.2016 JP 2016179185
(71) Applicant: Fujifilm Corporation, Minato-ku Tokyo 106-8620 (JP)
(72) Inventor: YAMAMOTO, Katsuya, Kanagawa 258-0023 (JP); IRISAWA, Kaku, Kanagawa 258-0023 (JP); MURAKOSHI, Dai, Kanagawa 258-0023 (JP)
(74) Representative: Klunker IP Patentanwälte PartG mbB
(86) International application number: PCT/JP2017/033339
(87) International publication number: WO 2018/052093

(57) **Abstract**

A photoacoustic image generation apparatus includes: a puncture needle (15) including a photoacoustic wave generation portion that absorbs light and generates photoacoustic waves; an acoustic wave detection unit (20) having a piezoelectric element array in which a plurality of piezoelectric elements that detect the photoacoustic waves are arranged; a control unit (28) that specifies some piezoelectric element groups in the piezoelectric element array and switches the piezoelectric element groups to acquire detection signals of all receiving regions in the piezoelectric element array; a photoacoustic image generation unit (24) that generates a photoacoustic image on the basis of the detection signals of the photoacoustic waves; and a tip position detection unit (29) that detects a position of a tip portion of the puncture needle (15) on the basis of the photoacoustic image. The control unit (28) specifies the piezoelectric element groups on the basis of the position of the tip portion of the puncture needle (15) detected by the tip position detection unit (29).

## Description

### BACKGROUND OF THE INVENTION

### 1. Field of the Invention

The present invention relates to a photoacoustic image generation apparatus comprising an insert of which at least a portion is inserted into a subject and which includes a photoacoustic wave generation portion that absorbs light and generates photoacoustic waves.

### 2. Description of the Related Art

An ultrasonography method has been known as a kind of image inspection method that can non-invasively inspect the internal state of a living body. In ultrasonography, an ultrasound probe that can transmit and receive ultrasonic waves is used. In a case in which the ultrasound probe transmits ultrasonic waves to a subject (living body), the ultrasonic waves travel in the living body and are reflected from the interface between tissues. The ultrasound probe receives the reflected ultrasonic waves and a distance is calculated on the basis of the time until the reflected ultrasonic waves return to the ultrasound probe. In this way, it is possible to capture an image indicating the internal aspect of the living body.

In addition, photoacoustic imaging has been known which captures the image of the inside of a living body using a photoacoustic effect. In general, in the photoacoustic imaging, the inside of the living body is irradiated with pulsed laser light. In the inside of the living body, a living body tissue absorbs the energy of the pulsed laser light and ultrasonic waves (photoacoustic waves) are generated by adiabatic expansion caused by the energy. For example, an ultrasound probe detects the photoacoustic waves and a photoacoustic image is formed on the basis of a detection signal. In this way, it is possible to visualize the inside of the living body on the basis of the photoacoustic waves.

In addition, as a technique related to the photoacoustic imaging, JP2015-231583A discloses a puncture needle in which a photoacoustic wave generation portion that absorbs light and generates photoacoustic waves is provided in the vicinity of a tip. In the puncture needle, an optical fiber is provided up to the tip of the puncture needle and light guided by the optical fiber is emitted to the photoacoustic wave generation portion. An ultrasound probe detects the photoacoustic waves generated by the photoacoustic wave generation portion and a photoacoustic image is generated on the basis of a detection signal of the photoacoustic waves. In the photoacoustic image, a part of the photoacoustic wave generation portion appears as a bight point, which makes it possible to check the position of the puncture needle using the photoacoustic image.

### SUMMARY OF THE INVENTION

Here, for example, in a case in which photoacoustic imaging is performed using the puncture needle disclosed in JP2015-231583A, the ultrasound probe detecting the photoacoustic waves comprises a piezoelectric element array in which a plurality of detection elements are arranged.

The piezoelectric element array of the ultrasound probe is formed by one-dimensionally arranging, for example, 128 piezoelectric elements and a receiving circuit receives a detection signal detected by the piezoelectric element through a multiplexer.

In general, the number of channels that can be received by the receiving circuit at the same time is limited. For example, in a case in which a 64-channel receiving circuit is used and 128 piezoelectric elements are provided as described above, the number of channels in the receiving circuit is less than the number of piezoelectric elements in the piezoelectric element array. Therefore, 128 detection elements are divided into detection element groups each of which include 64 detection elements and each of the divided detection element groups is selectively connected to the receiving circuit by a multiplexer with a ratio of 1:2.

However, for example, in a case in which a tip portion of the puncture needle is located at the boundary between the photoacoustic wave receiving regions of the detection element groups, it may be difficult to appropriately acquire a detection signal of the tip portion and to clearly display the tip portion of the puncture needle.

In addition, WO2012/008217A, JP2003-61955A, and JP1994-205773A (JP-H06-205773A) disclose a technique that adds and average overlap portions between a plurality of ultrasound images in order to make a joint between the ultrasound images inconspicuous, but do not disclose any technique for detecting the position of the tip portion of the puncture needle.

The invention has been made in view of the above-mentioned problems and an object of the invention is to provide a photoacoustic image generation apparatus that can appropriately acquire a detection signal of a tip portion of an insert, such as a puncture needle, and can more clearly display the tip portion of the insert.

A photoacoustic image generation apparatus according to the invention comprises: an insert of which at least a tip portion is inserted into a subject and which includes a light guide member that guides light to the tip portion and a photoacoustic wave generation portion that absorbs the light guided by the light guide member and generates photoacoustic waves; an acoustic wave detection unit having a detection element array in which a plurality of detection elements that detect the photoacoustic waves are arranged; a control unit that controls the acoustic wave detection unit to specify some detection element groups in the detection element array and switches the detection element groups to switch receiving regions of the photoacoustic waves, thereby acquiring detection signals of all of the receiving regions in the detection element array; a photoacoustic image generation unit that generates a photoacoustic image on the basis of the detection signals of the photoacoustic waves; and a tip position detection unit that detects a position of the tip portion of the insert on the basis of the photoacoustic image. The control unit specifies the detection element groups on the basis of the position of the tip portion of the insert detected by the tip position detection unit.

In the photoacoustic image generation apparatus according to the invention, the tip position detection unit may detect the position of the tip portion of the insert in time series on the basis of the photoacoustic image generated in time series and the control unit may sequentially specify the detection element groups on the basis of the position of the tip portion of the insert detected in time series.

In the photoacoustic image generation apparatus according to the invention, the control unit may store a plurality of switching patterns for the detection element groups in advance and the control unit may select any one of the plurality of switching patterns on the basis of the position of the tip portion of the insert and switch the detection element groups using the selected switching pattern.

In the photoacoustic image generation apparatus according to the invention, preferably, in a case in which the detection element array is formed by arranging the plurality of detection elements in at least one row and the arrangement direction is a left-right direction, one of the switching patterns for the detection element groups may be a pattern of switching two detection element groups, that is, a detection element group in a left half of the detection element array and a detection element group in a right half of the detection element array.

In the photoacoustic image generation apparatus according to the invention, one of the switching patterns for the detection element groups may be a pattern of switching two detection element groups, that is, a detection element group in a central portion of the detection element array and a detection element group on the left and right sides of the central portion of the detection element array and the control unit may select one of the pattern of switching the two detection element groups, that is, the detection element group in the left half of the detection element array and the detection element group in the right half of the detection element array and the pattern of switching the two detection element groups, that is, the detection element group in the central portion of the detection element array and the detection element group on the left and right sides of the central portion of the detection element array, on the basis of the position of the tip portion of the insert.

In the photoacoustic image generation apparatus according to the invention, preferably, a boundary between a receiving region of the detection element group in the left half of the detection element array and a receiving region the detection element group in the right half of the detection element array is included in a receiving region of the detection element group in the central portion of the detection element array.

In the photoacoustic image generation apparatus according to the invention, in a case in which a distance between the position of the tip portion of the insert and the boundary between the receiving regions of the detection element groups is equal to or less than a predetermined threshold value, the control unit may change the switching pattern.

In the photoacoustic image generation apparatus according to the invention, the control unit may alternately set the plurality of switching patterns before the position of the tip portion of the insert is detected and the tip position detection unit may detect the position of the tip portion of the insert on the basis of each photoacoustic image corresponding to each of the switching patterns.

In the photoacoustic image generation apparatus according to the invention, in a case in which the tip position detection unit is not capable of detecting the position of the tip portion of the insert, the control unit may alternately set the plurality of switching patterns and the tip position detection unit may detect the position of the tip portion of the insert on the basis of each photoacoustic image corresponding to each of the switching patterns.

The photoacoustic image generation apparatus according to the invention may further comprise a switching pattern selection receiving unit that receives a selection of the switching pattern initially set before the position of the tip portion of the insert is detected.

In the photoacoustic image generation apparatus according to the invention, the switching pattern selection receiving unit may receive a selection of a highlighting process for the insert as the selection of the initially set switching pattern.

In the photoacoustic image generation apparatus according to the invention, preferably, the insert is a needle that is inserted into the subject.

The photoacoustic image generation apparatus according to the invention controls the acoustic wave detection unit having the detection element array to specify some detection element groups in the detection element array, switches some detection element groups to switch photoacoustic wave receiving regions, thereby acquiring the detection signals of all of the receiving regions in the detection element array, and generates a photoacoustic image on the basis of the detection signals.

Then, the photoacoustic image generation apparatus detects the position of the tip portion of the insert having the photoacoustic wave generation portion on the basis of the photoacoustic image and specifies some detection element groups on the basis of the detected position of the tip portion of the insert. Therefore, it is possible to appropriately acquire the detection signal of the tip portion of the insert and to clearly display the tip portion of the insert.

### BRIEF DESCRIPTION OF THE DRAWINGS

Fig. 1 is a block diagram schematically illustrating the configuration of a first embodiment of a photoacoustic image generation apparatus according to the invention.
Fig. 2 is a cross-sectional view illustrating the configuration of a tip portion of a puncture needle.
Fig. 3 is a block diagram schematically illustrating the configuration of an acoustic wave detection unit.
Fig. 4 is a flowchart illustrating a method for specifying a piezoelectric element group in the photoacoustic image generation apparatus according to the first embodiment.
Fig. 5 is a diagram illustrating an example of a piezoelectric element group switching pattern.
Fig. 6 is a diagram illustrating the method for specifying the piezoelectric element group in the photoacoustic image generation apparatus according to the first embodiment.
Fig. 7 is a diagram illustrating another example of the piezoelectric element group switching pattern.
Fig. 8 is a flowchart illustrating a method for specifying a piezoelectric element group in a photoacoustic image generation apparatus according to a second embodiment.
Fig. 9 is a diagram illustrating the method for specifying the piezoelectric element group in the photoacoustic image generation apparatus according to the second embodiment.
Fig. 10 is a diagram illustrating an example of four switching patterns used in the photoacoustic image generation apparatus according to the second embodiment.
Fig. 11 is a block diagram schematically illustrating another embodiment of the photoacoustic image generation apparatus according to the invention.
Fig. 12 is a flowchart illustrating a method for specifying an initially set switching pattern on the basis of the selection of a needle highlighting process.

### DESCRIPTION OF THE PREFERRED EMBODIMENTS

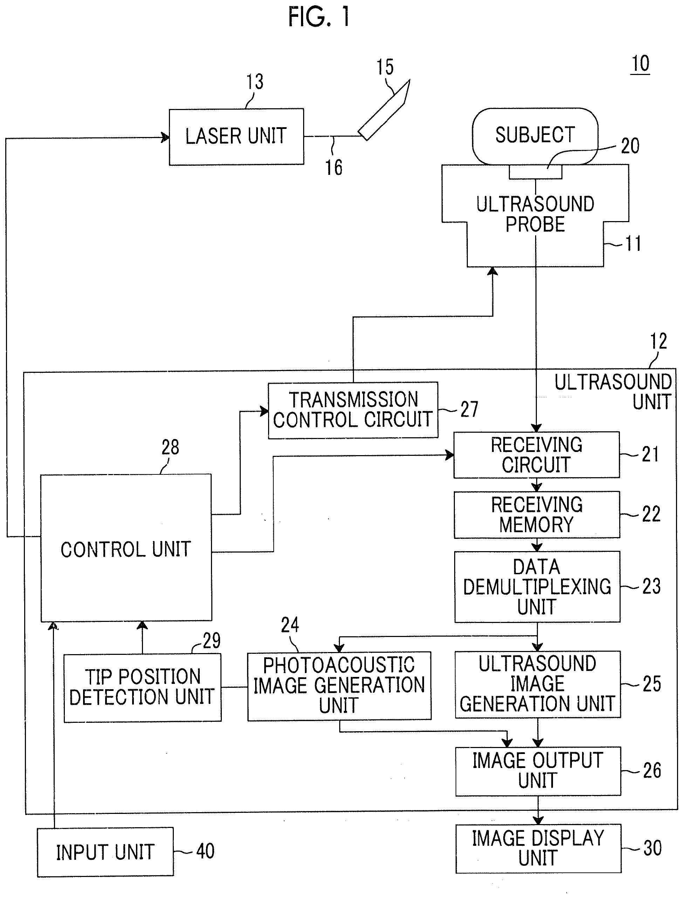

Hereinafter, a first embodiment of a photoacoustic image generation apparatus according to the invention will be described in detail with reference to the drawings. Fig. 1 is a diagram schematically illustrating the configuration of a photoacoustic image generation apparatus 10 according to this embodiment.

As illustrated in Fig. 1, the photoacoustic image generation apparatus 10 according to this embodiment comprises an ultrasound probe 11, an ultrasound unit 12, a laser unit 13, and a puncture needle 15. The puncture needle 15 and the laser unit 13 are connected by an optical cable 16 having an optical fiber. The puncture needle 15 can be attached to and detached from the optical cable 16 and is disposable. In addition, in this embodiment, ultrasonic waves are used as acoustic waves. However, the invention is not limited to the ultrasonic waves. Acoustic waves with an audible frequency may be used as long as an appropriate frequency can be selected according to, for example, an inspection target or measurement conditions.

The laser unit 13 comprises a solid-state laser light source using, for example, yttrium aluminum garnet (YAG) and alexandrite. Laser light emitted from the solid-state laser light source of the laser unit 13 is guided by the optical cable 16 and is incident on the puncture needle 15. The laser unit 13 according to this embodiment emits pulsed laser light in a near-infrared wavelength range. The near-infrared wavelength range means a wavelength range from 700 nm to 850 nm. In this embodiment, the solid-state laser light source is used. However, other laser light sources, such as a gas laser light source, may be used or light sources other than the laser light source may be used.

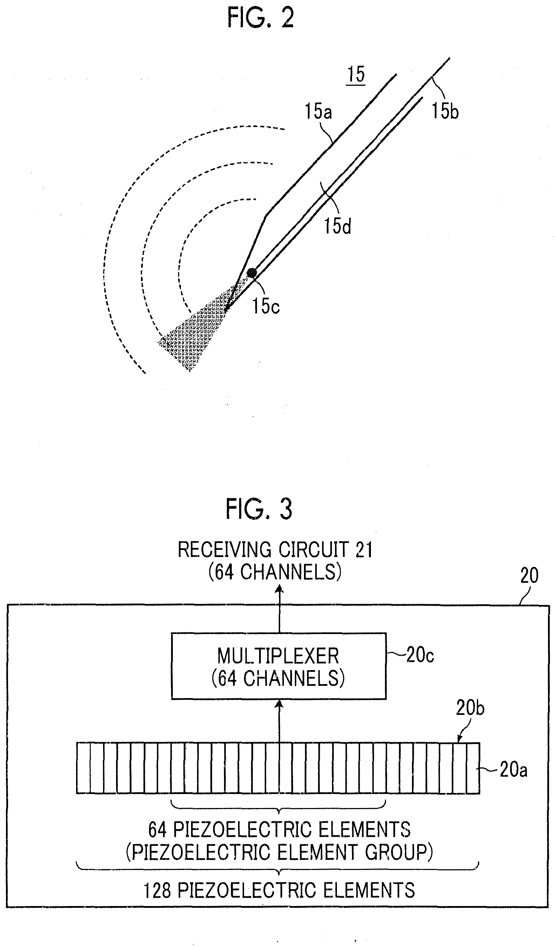

The puncture needle 15 is an embodiment of an insert according to the invention and is a needle that is inserted into a subject. Fig. 2 is a cross-sectional view including a center axis that extends in a length direction of the puncture needle 15. The puncture needle 15 includes a puncture needle main body 15a that has an opening at an acute tip and is formed in a hollow shape, an optical fiber 15b (corresponding to a light guide member according to the invention) that guides laser light emitted from the laser unit 13 to the vicinity of the opening of the puncture needle 15, and a photoacoustic wave generation portion 15c that absorbs laser light emitted from the optical fiber 15b and generates photoacoustic waves.

The optical fiber 15b and the photoacoustic wave generation portion 15c are provided in a hollow portion 15d of the puncture needle main body 15a. For example, the optical fiber 15b is connected to the optical fiber in the optical cable 16 (see Fig. 1) through an optical connector that is provided at the base end of the puncture needle 15. For example, a laser light of 0.2 mJ is emitted from a light emission end of the optical fiber 15b.

The photoacoustic wave generation portion 15c is provided at the light emission end of the optical fiber 15b and is provided in the vicinity of the tip of the puncture needle 15 and in the inner wall of the puncture needle main body 15a. The photoacoustic wave generation portion 15c absorbs the laser light emitted from the optical fiber 15b and generates photoacoustic waves. The photoacoustic wave generation portion 15c is made of, for example, an epoxy resin, a polyurethane resin, a fluorine resin, and silicone rubber with which a black pigment is mixed. In Fig. 2, the photoacoustic wave generation portion 15c is illustrated to be larger than the optical fiber 15b. However, the invention is not limited thereto. The photoacoustic wave generation portion 15c may have a size that is equal to the diameter of the optical fiber 15b.

The photoacoustic wave generation portion 15c is not limited to the above and a metal film or an oxide film having light absorptivity with respect to the wavelength of laser light may be used as the photoacoustic wave generation portion. An oxide film made of, for example, iron oxide, chromium oxide, or manganese oxide having high light absorptivity with respect to the wavelength of laser light can be used as the photoacoustic wave generation portion 15c. Alternatively, a metal film made of, for example, titanium (Ti) or platinum (Pt) that has a lower light absorptivity than an oxide and has a higher biocompatibility than an oxide may be used as the photoacoustic wave generation portion 15c. In addition, the position where the photoacoustic wave generation portion 15c is provided is not limited to the inner wall of the puncture needle main body 15a. For example, a metal film or an oxide film which is the photoacoustic wave generation portion 15c may be formed on the light emission end of the optical fiber 15b with a thickness of about 100 nm by vapor deposition such that the oxide film covers the light emission end. In this case, at least a portion of the laser light emitted from the light emission end of the optical fiber 15b is absorbed by the metal film or the oxide film covering the light emission end and photoacoustic waves are generated from the metal film or the oxide film.

The vicinity of the tip of the puncture needle 15 means a position where the photoacoustic wave generation portion 15c can generate photoacoustic waves capable of imaging the position of the tip of the puncture needle 15 with accuracy required for a needling operation in a case in which the tip of the optical fiber 15b and the photoacoustic wave generation portion 15c are disposed at the position. For example, the vicinity of the tip of the puncture needle 15 is the range of 0 mm to 3 mm from the tip to the base end of the puncture needle 15. In the subsequent embodiments, the meaning of the vicinity of the tip is the same as described above.

Returning to Fig. 1, the ultrasound probe 11 detects the photoacoustic waves emitted from the photoacoustic wave generation portion 15c after the puncture needle 15 is inserted into the subject. The ultrasound probe 11 includes an acoustic wave detection unit 20 that detects photoacoustic waves.

As illustrated in Fig. 3, the acoustic wave detection unit 20 comprises a piezoelectric element array 20b (corresponding to a detection element array according to the invention) in which a plurality of piezoelectric elements 20a (corresponding to detection elements according to the invention) that detect photoacoustic waves are one-dimensionally arranged and a multiplexer 20c. The piezoelectric element 20a is an ultrasound transducer and is, for example, a piezoelectric element made of a polymer film, such as piezoelectric ceramics or polyvinylidene fluoride (PVDF). In addition, the acoustic wave detection unit 20 comprises, for example, an acoustic lens, an acoustic matching layer, a backing material, and a control circuit for the piezoelectric element array 20b which are not illustrated in the drawings.

The piezoelectric element array 20b according to this embodiment comprises 128 piezoelectric elements 20a. The arrangement and number of piezoelectric elements 20a are not limited thereto. For example, the number of piezoelectric elements 20a may be 256 or the piezoelectric elements 20a may be two-dimensionally arranged. In the specification, the arrangement direction of the piezoelectric elements may be a one-dimensional direction in a case in which the piezoelectric elements are one-dimensionally arranged and may be one of two directions perpendicular to each other in a two-dimensional space in a case in which the piezoelectric elements are two-dimensionally arranged. It is preferable that the arrangement direction is a direction in which a large number of piezoelectric elements are arranged.

The multiplexer 20c selectively connects some piezoelectric element groups that detect photoacoustic waves in parallel among the piezoelectric elements 20a forming the piezoelectric element array 20b to a receiving circuit 21 of the ultrasound unit 12. The piezoelectric element group is a set of the piezoelectric elements 20a that detect photoacoustic waves in parallel.

A photoacoustic wave receiving region of the piezoelectric element array 20b is divided into a plurality of receiving regions by the selective connection by the multiplexer 20c and a photoacoustic wave detection signal is acquired from each receiving region. In addition, the photoacoustic wave receiving region is a region of the subject from which photoacoustic waves can be received by the piezoelectric element groups.

Specifically, the multiplexer 20c according to this embodiment has 64 channels and can acquire the detection signals of 64 piezoelectric element groups in parallel. 64 piezoelectric element groups connected to the receiving circuit 21 by the multiplexer 20c are specified by a control unit 28 of the ultrasound unit 12. The control unit 28 switches 64 piezoelectric element groups to switch the photoacoustic wave receiving regions, thereby acquiring the detection signals of all of the receiving regions of the piezoelectric element array 20b.

The ultrasound probe 11 performs the transmission of acoustic waves (ultrasonic waves) to the subject and the reception of reflected acoustic waves (reflected ultrasonic waves) with respect to the transmitted ultrasonic waves, in addition to the detection of the photoacoustic waves, using the piezoelectric element array 20b of the acoustic wave detection unit 20. The transmission and reception of the ultrasonic waves may be performed at different positions. For example, ultrasonic waves may be transmitted from a position different from the position of the ultrasound probe 11 and the piezoelectric element array 20b of the ultrasound probe 11 may receive the reflected ultrasonic waves with respect to the transmitted ultrasonic waves. For example, a linear ultrasound probe, a convex ultrasound probe, or a sector ultrasound probe may be used as the ultrasound probe 11.

The ultrasound unit 12 includes the receiving circuit 21, a receiving memory 22, a data demultiplexing unit 23, a photoacoustic image generation unit 24, an ultrasound image generation unit 25, an image output unit 26, a transmission control circuit 27, the control unit 28, and a tip position detection unit 29. The ultrasound unit 12 typically includes, for example, a processor, a memory, and a bus. A program related to, for example, a photoacoustic image generation process, an ultrasound image generation process, and a process of detecting the position of the tip of the puncture needle 15 in a photoacoustic image is incorporated into a memory in the ultrasound unit 12. The program is executed by the control unit 28 which is formed by a processor to implement the functions of the data demultiplexing unit 23, the photoacoustic image generation unit 24, the ultrasound image generation unit 25, the image output unit 26, and the tip position detection unit 29. That is, each of these units is formed by the processor and the memory into which the program has been incorporated.

The hardware configuration of the ultrasound unit 12 is not particularly limited and can be implemented by an appropriate combination of, for example, a plurality of integrated circuits (ICs), a processor, an application specific integrated circuit (ASIC), a field-programmable gate array (FPGA), and a memory.

The receiving circuit 21 receives a detection signal output from the ultrasound probe 11 and stores the received detection signal in the receiving memory 22. The receiving circuit 21 according to this embodiment has 64 channels. The receiving circuit 21 typically includes a low-noise amplifier, a variable-gain amplifier, a low-pass filter, and an analog-to-digital converter (AD converter). The detection signal of the ultrasound probe 11 is amplified by the low noise amplifier. Then, gain adjustment corresponding to a depth is performed by the variable-gain amplifier and a high-frequency component of the detection signal is cut by the low-pass filter. Then, the detection signal is converted into a digital signal by the AD converter and the digital signal is stored in the receiving memory 22. The receiving circuit 21 is formed by, for example, one integral circuit (IC).

The ultrasound probe 11 outputs a detection signal of the photoacoustic waves and a detection signal of the reflected ultrasonic waves. The AD-converted detection signals (sampling data) of the photoacoustic waves and the reflected ultrasonic waves are stored in the receiving memory 22. The data demultiplexing unit 23 reads the detection signal of the photoacoustic waves from the receiving memory 22 and transmits the detection signal to the photoacoustic image generation unit 24. In addition, the data demultiplexing unit 23 reads the detection signal of the reflected ultrasonic waves from the receiving memory 22 and transmits the detection signal to the ultrasound image generation unit 25.

The photoacoustic image generation unit 24 generates a photoacoustic image on the basis of the detection signal of the photoacoustic waves detected by the ultrasound probe 11. The photoacoustic image generation process includes, for example, image reconfiguration, such as phasing addition, detection, and logarithmic conversion. The ultrasound image generation unit 25 generates an ultrasound image (reflected acoustic image) on the basis of the detection signal of the reflected ultrasonic waves detected by the ultrasound probe 11. The ultrasound image generation process includes, for example, image reconfiguration, such as phasing addition, detection, and logarithmic conversion. The image output unit 26 outputs the photoacoustic image and the ultrasound image to an image display unit 30 such as a display device.

The tip position detection unit 29 detects the position of a tip portion of the puncture needle 15 on the basis of the photoacoustic image generated by the photoacoustic image generation unit 24. As a method for detecting the position of the tip portion of the puncture needle 15, any method may be used as long as it can detect the position of a maximum brightness point in the photoacoustic image as the position of the tip portion of the puncture needle 15.

In a case in which the position of the tip of the puncture needle 15 is detected on the basis of the photoacoustic image as described above, in practice, an artifact of light or an artifact of sound is generated and a photoacoustic image in which photoacoustic waves are detected from a plurality of positions is likely to be generated and the original position of the tip portion of the puncture needle 15 is unlikely to be specified.

For this reason, the photoacoustic image generated by the photoacoustic image generation unit 24 is not used as it is, but, for example, a smoothing process may be performed for the photoacoustic image to prevent erroneous detection caused by the artifact. Specifically, the smoothing process is performed for the photoacoustic image subjected to detection and logarithmic conversion. For example, a filtering process using a Gaussian filter can be used as the smoothing process. It is preferable that the size of the Gaussian filter is less than that of the tip portion of the puncture needle 15.

Then, a binarization process is performed for the photoacoustic image subjected to the smoothing process to generate a binary image. Then, a region in which white pixels are continuously distributed is detected from the binary image to detect the position of the tip portion of the puncture needle 15. In this way, it is possible to detect the position of the tip portion of the puncture needle 15 with higher accuracy.

The control unit 28 controls each component in the ultrasound unit 12. For example, in a case in which a photoacoustic image is acquired, the control unit 28 transmits a trigger signal to the laser unit 13 such that the laser unit 13 emits laser light. In addition, the control unit 28 transmits a sampling trigger signal to the receiving circuit 21 to control, for example, the sampling start time of the photoacoustic waves with the emission of the laser light.

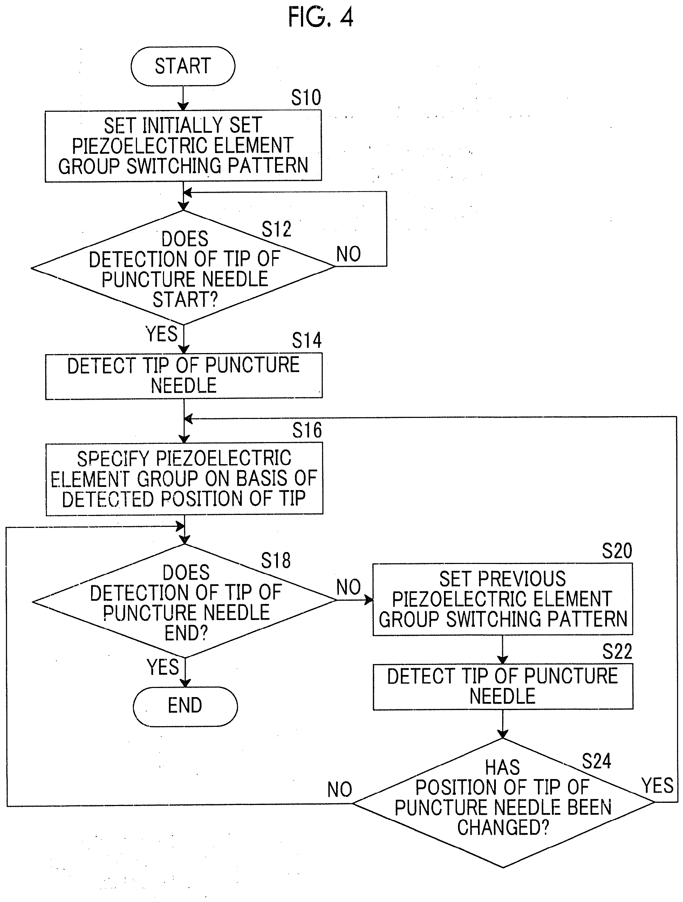

Here, the control unit 28 according to this embodiment controls the multiplexer 20c of the acoustic wave detection unit 20 to specify 64 piezoelectric element groups connected to the receiving circuit 21 as described above. At that time, the control unit 28 specifies 64 piezoelectric element groups (hereinafter, simply referred to as piezoelectric element groups) on the basis of the position of the tip portion of the puncture needle 15 detected by the tip position detection unit 29. Specifically, the control unit 28 according to this embodiment sequentially specifies the piezoelectric element groups in real time, following a change in the position of the tip portion of the puncture needle 15, such that the position of the tip portion of the puncture needle 15 is always located at the center of the receiving region of the piezoelectric element group. Next, a method for specifying the piezoelectric element group will be described with reference to a flowchart illustrated in Fig. 4.

First, the control unit 28 sets the initially set piezoelectric element group switching pattern that has been stored in advance (S10). In this embodiment, a pattern of switching a piezoelectric element group in the left half of the piezoelectric element array 20b and a piezoelectric element group in the right half of the piezoelectric element array 20b is set as the initially set piezoelectric element group switching pattern. Here, it is assumed that the arrangement direction of the piezoelectric elements 20a is a left-right direction. In addition, the initially set piezoelectric element group switching pattern is not limited thereto and may be other patterns.

Then, the control unit 28 checks whether the user has input a command to start the detection of the tip of the puncture needle 15. In a case in which the tip detection start command has been input (S12, YES), the control unit 28 starts a process of detecting the position of the tip portion of the puncture needle 15 (S14). In addition, the user inputs the tip detection start command and a tip detection end command with an input unit 40 (see Fig. 1).

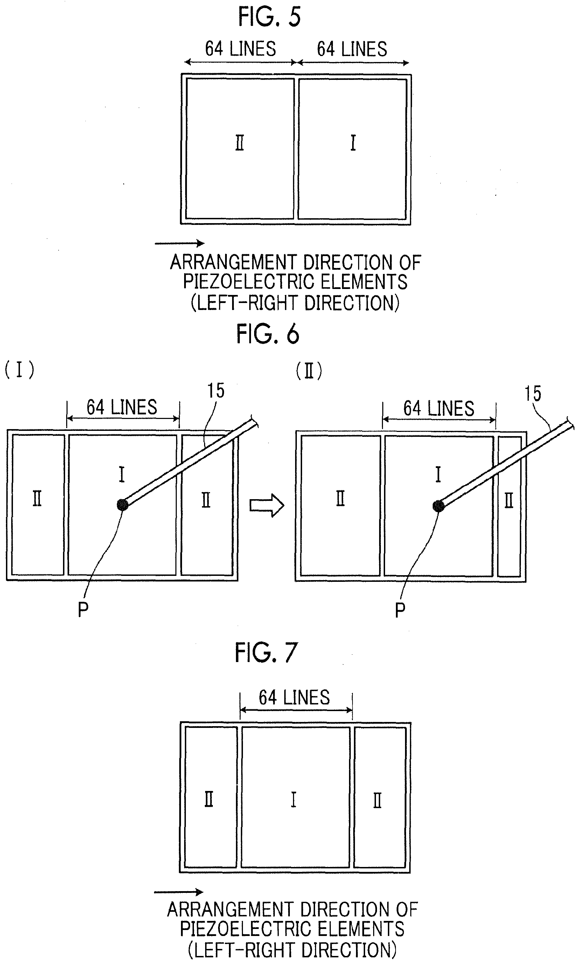

Specifically, the control unit 28 switches the piezoelectric element group in the left half and the piezoelectric element group in the right half to switch a receiving region I of the piezoelectric element group in the right half and a receiving region II of the piezoelectric element group in the left half illustrated in Fig. 5, thereby acquiring the detection signals of the photoacoustic waves from all of the receiving regions. That is, first, the control unit 28 acquires a detection signal of the receiving region I of the piezoelectric element group in the right half and stores the detection signal in the receiving memory 22. Then, the control unit 28 acquires a detection signal of the receiving region II of the piezoelectric element group in the left half and stores the detection signal in the receiving memory 22. Each of 64 lines illustrated in Fig. 5 is a region of the subject from which a detection signal is acquired by phasing addition centering on each piezoelectric element.

Then, the data demultiplexing unit 23 transmits the detection signals of all of the receiving regions from the receiving memory 22 to the photoacoustic image generation unit 24 and the photoacoustic image generation unit 24 generates a photoacoustic image corresponding to one frame.

A photoacoustic image of one frame generated by the photoacoustic image generation unit 24 is input to the tip position detection unit 29. The tip position detection unit 29 detects the position of the tip portion of the puncture needle 15.

The positional information of the tip portion detected by the tip position detection unit 29 is input to the control unit 28 and the control unit 28 specifies the piezoelectric element group used in a case in which a photoacoustic image of the next frame is acquired, on the basis of the input positional information of the tip portion (S16). Specifically, for example, in a case in which a tip portion P of the puncture needle 15 is located at a position illustrated in (I) of Fig. 6, the control unit 28 specifies a receiving region I in which the tip portion P is located at the center and specifies a piezoelectric element group corresponding to the receiving region I. Then, the control unit 28 acquires a detection signal of the receiving region I of the specified piezoelectric element group in the receiving memory 22. Then, the control unit 28 acquires a detection signal of the other receiving region II and stores the detection signal in the receiving memory 22.

Then, the detection signals of all of the receiving regions are transmitted from the receiving memory 22 to the photoacoustic image generation unit 24. The photoacoustic image generation unit 24 generates a photoacoustic image of one frame. The image output unit 26 displays the photoacoustic image of one frame on the image display unit 30. In this embodiment, as described above, the piezoelectric element group corresponding to the receiving region I in which the tip portion P of the puncture needle 15 is located at the center is specified. Therefore, it is possible to appropriately acquire the detection signal of the tip portion P of the puncture needle 15 and an image of the tip portion P of the puncture needle 15 is clearly displayed on a photoacoustic image.

Then, the control unit 28 checks whether the user has input a command to end the detection of the tip of the puncture needle 15 (S18). In a case in which the tip detection end command has not been input, the control unit 28 sets the previously set piezoelectric element group switching pattern (S20).

That is, the receiving region I and the receiving region II illustrated in (I) of Fig. 6 are set again. The detection signal of the receiving region I is acquired and then the detection signal of the receiving region II are acquired. Then, a photoacoustic image based on the detection signals is input to the tip position detection unit 29 and the position of the tip portion of the puncture needle 15 is detected (S22).

Then, in a case in which the position of the tip portion of the puncture needle 15 has not been changed and the tip detection end command has not been input (S24, NO and S18, NO), the receiving region I and the receiving region II are set again (S20) and the position of the tip portion of the puncture needle 15 is detected again (S22). In contrast, in a case in which the position of the tip portion of the puncture needle 15 has been changed as illustrated in (II) of Fig. 6 (S24, YES), the process returns to S16. A receiving region I having the changed position of the tip portion P at the center is specified and a piezoelectric element group corresponding to the receiving region I is specified.

Then, the detection signal of the receiving region I of the specified piezoelectric element group is acquired and stored in the receiving memory 22. Then, the detection signal of the other receiving region II is acquired and stored in the receiving memory 22.

Then, the detection signals of all of the receiving regions are transmitted from the receiving memory 22 to the photoacoustic image generation unit 24 and the photoacoustic image generation unit 24 generates a photoacoustic image corresponding to one frame. The image output unit 26 displays the photoacoustic image of one frame on the image display unit 30.

Then, the process from S16 to S24 is repeatedly performed. In a case in which the tip detection end command is input in S18, the process ends.

As described above, in this embodiment, the tip position detection unit 29 detects the position of the tip portion of the puncture needle 15 in time series on the basis of the photoacoustic image generated in time series and the control unit 28 sequentially specifies the piezoelectric element groups on the basis of the position of the tip portion of the puncture needle 15 detected in time series.

As such, since the piezoelectric element groups are sequentially specified on the basis of the position of the tip portion of the puncture needle 15, it is possible to appropriately acquire the detection signal of the tip portion of the puncture needle 15 and to more clearly display the tip portion of the puncture needle 15 on a photoacoustic image.

Then, in a case in which an ultrasound image is acquired, the control unit 28 transmits an ultrasonic wave transmission trigger signal for commanding the transmission of ultrasonic waves to the transmission control circuit 27. In a case in which the ultrasonic wave transmission trigger signal is received, the transmission control circuit 27 directs the ultrasound probe 11 to transmit ultrasonic waves. In a case in which an ultrasound image is acquired, for example, the ultrasound probe 11 scans the receiving region of the piezoelectric element group line by line to detect the reflected ultrasonic waves under the control of the control unit 28. The control unit 28 transmits a sampling trigger signal to the receiving circuit 21 according to an ultrasonic wave transmission time to start the sampling of the reflected ultrasonic waves. Sampling data received by the receiving circuit 21 is stored in the receiving memory 22.

The ultrasound image generation unit 25 receives the sampling data of the detection signal of the reflected ultrasonic waves through the data demultiplexing unit 23 and generates an ultrasound image. The ultrasound image generated by the ultrasound image generation unit 25 is input to the image output unit 26 and the image output unit 26 displays the ultrasound image on the image display unit 30.

In the image display unit 30, the photoacoustic image and the ultrasound image may be separately displayed or may be combined and displayed. In a case in which the photoacoustic image and the ultrasound image are combined and displayed, it is possible to check the position of the tip of the puncture needle 15 in a living body and thus to perform accurate and safe needling.

Next, a second embodiment of the photoacoustic image generation apparatus according to the invention will be described. In the photoacoustic image generation apparatus 10 according to the first embodiment, the piezoelectric element groups are sequentially specified in real time, following a change in the position of the tip portion of the puncture needle 15, such that the tip portion of the puncture needle 15 is always located at the center of the receiving region of the piezoelectric element group. However, in a photoacoustic image generation apparatus 10 according to the second embodiment, a plurality of piezoelectric element group switching patterns are stored in advance and any one of the plurality of switching patterns is selected on the basis of the position of the tip portion of the puncture needle 15. The other configurations and operations are the same as those in the photoacoustic image generation apparatus 10 according to the first embodiment.

In addition to the switching pattern illustrated in Fig. 5, a switching pattern illustrated in Fig. 7 is stored in advance in the control unit 28 of the photoacoustic image generation apparatus 10 according to the second embodiment. The switching pattern illustrated in Fig. 7 is a pattern of switching two piezoelectric element groups, that is, a piezoelectric element group in a central portion of the piezoelectric element array 20b and a piezoelectric element group on the left and right sides of the central portion. In addition, the boundary between the receiving region I of the piezoelectric element group in the right half and the receiving region II of the piezoelectric element group in the left half illustrated in Fig. 5 is set so as to be included in a receiving region I of the piezoelectric element group in the central portion illustrated in Fig. 7.

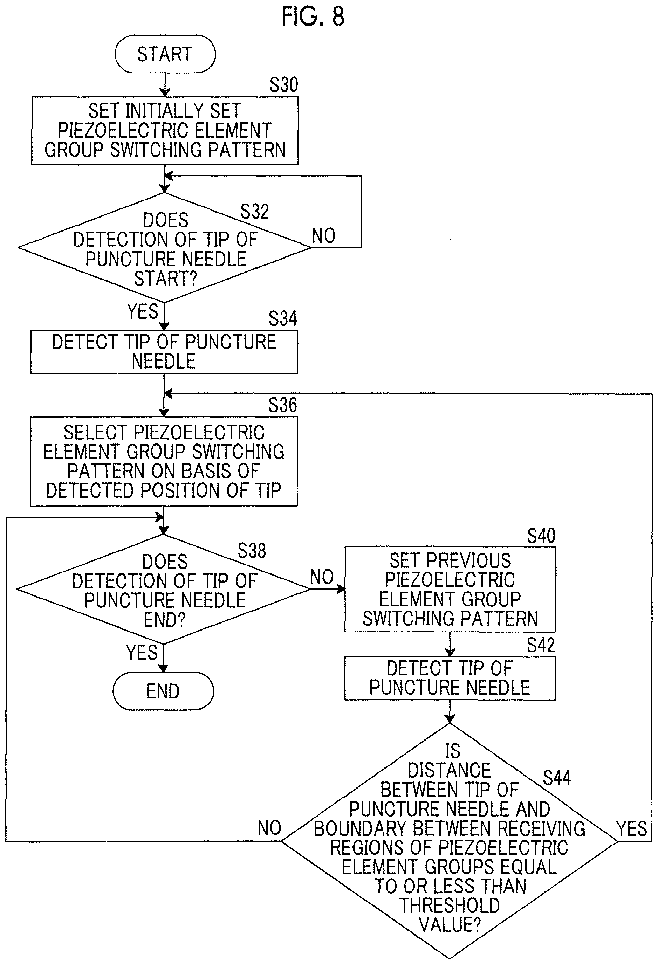

Then, the control unit 28 selects one of the switching pattern illustrated in Fig. 5 and the switching pattern illustrated in Fig. 7 on the basis of the position of the tip portion of the puncture needle 15 and sets the selected switching pattern. Next, a piezoelectric element group specification method according to the second embodiment will be described with reference to a flowchart illustrated in Fig. 8.

First, the control unit 28 sets the initially set piezoelectric element group switching pattern that has been stored in advance, as in the first embodiment (S30). In this embodiment, the switching pattern illustrated in Fig. 5 is set as the initially set piezoelectric element group switching pattern. In the second embodiment, the initially set piezoelectric element group switching pattern is not limited thereto and may be other patterns.

Then, the control unit 28 checks whether the user has input a command to start the detection of the tip of the puncture needle 15. In a case in which the tip detection start command has been input (S32, YES), the control unit 28 starts a process of detecting the position of the tip portion of the puncture needle 15 (S34).

Specifically, the control unit 28 switches the piezoelectric element group in the left half and the piezoelectric element group in the right half to switch the receiving region I of the piezoelectric element group in the right half and the receiving region II of the piezoelectric element group in the left half illustrated in Fig. 5, thereby acquiring the detection signals of all of the receiving regions. That is, first, the control unit 28 acquires the detection signal of the receiving region I of the piezoelectric element group in the right half and stores the detection signal in the receiving memory 22. Then, the control unit 28 acquires the detection signal of the receiving region II of the piezoelectric element group in the left half and stores the detection signals in the receiving memory 22.

Then, the data demultiplexing unit 23 transmits the detection signals of all of the receiving regions from the receiving memory 22 to the photoacoustic image generation unit 24 and the photoacoustic image generation unit 24 generates a photoacoustic image corresponding to one frame.

The photoacoustic image of one frame generated by the photoacoustic image generation unit 24 is input to the tip position detection unit 29. The tip position detection unit 29 detects the position of the tip portion of the puncture needle 15 (S34).

The positional information of the tip portion detected by the tip position detection unit 29 is input to the control unit 28 and the control unit 28 selects a piezoelectric element group switching pattern used in a case in which a photoacoustic image of the next frame is acquired, on the basis of the input positional information of the tip portion (S36).

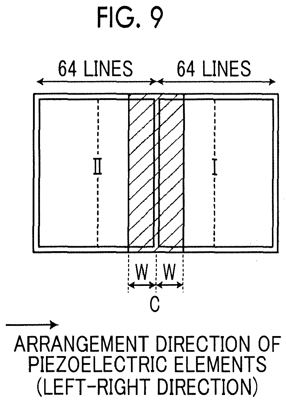

Specifically, for example, in a case in which the position of the tip portion P of the puncture needle 15 is in a hatched range illustrated in Fig. 9, the switching pattern illustrated in Fig. 7 is selected. In a case in which the position is out of the hatched range illustrated in Fig. 9, the switching pattern illustrated in Fig. 5 is continuously selected and set. The hatched range illustrated in Fig. 9 includes a boundary C between the receiving region I of the piezoelectric element group in the right half and the receiving region II of the piezoelectric element group in the left half illustrated in Fig. 5 and has a width of W on the left and right sides of the boundary C as a center line.

The width of W is preset by the user. In Fig. 9, the boundary between the receiving region I and the receiving region II in the switching pattern illustrated in Fig. 7 is represented by a dotted line. The width of W is set so as not to include the boundary between the receiving region I and the receiving region II in the switching pattern illustrated in Fig. 7.

Then, the detection signal of the receiving region I of the selected switching pattern is acquired and stored in the receiving memory 22. Then, the detection signal of the other receiving region II is acquired and stored in the receiving memory 22.

The detection signals of all of the receiving regions are transmitted from the receiving memory 22 to the photoacoustic image generation unit 24. The photoacoustic image generation unit 24 generates a photoacoustic image of one frame and the image output unit 26 displays the photoacoustic image of one frame on the image display unit 30. In this embodiment, the piezoelectric element group switching pattern is selected such that the tip portion P of the puncture needle 15 is not included in a boundary portion between the receiving regions. Therefore, it is possible to appropriately acquire the detection signal of the tip portion P of the puncture needle 15 and the image of the tip portion P of the puncture needle 15 is clearly displayed on a photoacoustic image.

Then, the control unit 28 checks whether the user has input a command to end the detection of the tip of the puncture needle 15 (S38). In a case in which the tip detection end command has not been input, the control unit 28 sets the previously set piezoelectric element group switching pattern (S40).

That is, after the detection signal of the receiving region I is acquired again, the detection signal of the receiving region II is acquired and a photoacoustic image based on the detection signals is input to the tip position detection unit 29. Then, the position of the tip portion of the puncture needle 15 is detected (S42).

Then, in a case in which a distance between the position of the tip portion of the puncture needle 15 and the boundary between the receiving region I and the receiving region II in the currently set switching pattern is equal to or less than a threshold value (S44, YES), the process returns to S36 and the switching pattern is changed. Specifically, in this embodiment, in a case in which the currently set switching pattern is the switching pattern illustrated in Fig. 5 and the position of the tip portion P of the puncture needle 15 is within the hatched range illustrated in Fig. 9, the switching pattern is changed to the switching pattern illustrated in Fig. 7. On the other hand, in a case in which the currently set switching pattern is the switching pattern illustrated in Fig. 7 and the position of the tip portion P of the puncture needle 15 is changed to be out of the hatched range illustrated in Fig. 9, the switching pattern is changed to the switching pattern illustrated in Fig. 5.

In contrast, in a case in which the distance between the position of the tip portion of the puncture needle 15 and the boundary between the receiving region I and the receiving region II in the currently set switching pattern is greater than the threshold value and the tip detection end command has not been input (S44, NO, and S38, NO), the same receiving regions I and II are set again (S40) and the position of the tip portion of the puncture needle 15 is detected again (S42). Then, the detection signal of the set receiving region I is acquired and stored in the receiving memory 22. Then, the detection signal of the other receiving region II is acquired and stored in the receiving memory 22.

Then, the detection signals of all of the receiving regions are transmitted from the receiving memory 22 to the photoacoustic image generation unit 24 and the photoacoustic image generation unit 24 generates a photoacoustic image corresponding to one frame. The image output unit 26 displays the photoacoustic image corresponding to one frame on the image display unit 30.

Then, the process from S36 to S44 is repeatedly performed. In a case in which the tip detection end command is input in S38, the process ends.

In the photoacoustic image generation apparatus 10 according to the second embodiment, a plurality of preset switching patterns are selected on the basis of the position of the tip portion of the puncture needle 15 and are then used. Therefore, it is possible to simplify the process and thus to increase a processing speed, as compared to a case in which the piezoelectric element groups are sequentially specified according to the position of the tip portion of the puncture needle 15 as in the photoacoustic image generation apparatus 10 according to the first embodiment.

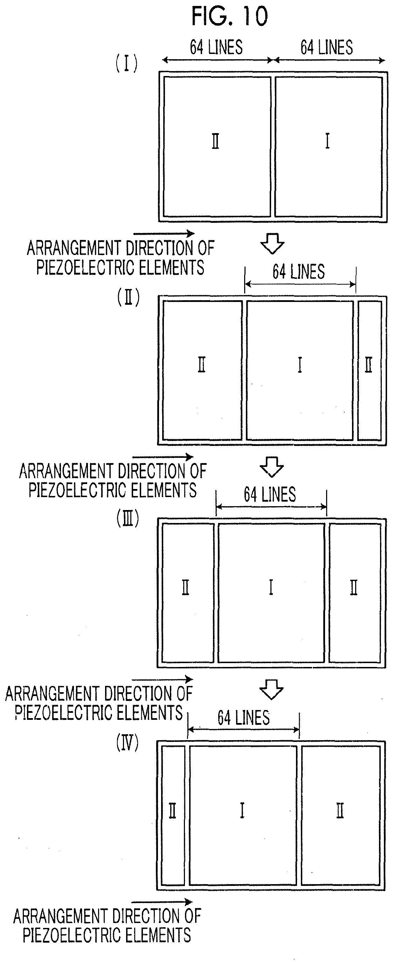

Further, in the photoacoustic image generation apparatus 10 according to the second embodiment, two switching patterns are used. However, the invention is not limited thereto. For example, three or more switching patterns may be used. Fig. 10 illustrates four switching patterns. (I) of Fig. 10 illustrates a pattern of switching a piezoelectric element group in the right half and a piezoelectric element group in the left half, similarly to the switching pattern illustrated in Fig. 5. (III) of Fig. 10 illustrates a pattern of switching two piezoelectric element groups, that is, a piezoelectric element group in a central portion of the piezoelectric element array 20b and a piezoelectric element group on the left and right sides of the central portion, similarly to the switching pattern illustrated in Fig. 7. In addition, (II) of Fig. 10 illustrates a switching pattern in which a receiving region I including both the boundary between the receiving regions in the switching pattern illustrated in (I) of Fig. 10 and the right boundary between the receiving regions in the switching pattern illustrated in (III) of Fig. 10 and a receiving region II on the left and right sides of the receiving region I are set. (IV) of Fig. 10 illustrates a switching pattern in which a receiving region I including both the boundary between the receiving regions in the switching pattern illustrated in (I) of Fig. 10 and the left boundary between the receiving regions in the switching pattern illustrated in (III) of Fig. 10 and a receiving region II on the left and right sides of the receiving region I are set. The receiving region I in the switching pattern illustrated in (II) of Fig. 10 is set such that the center thereof is located on the right side of the center of the piezoelectric element array 20b. The center of the receiving region I in the switching pattern illustrated in (IV) of Fig. 10 is set such that the center thereof is located on the left side of the center of the piezoelectric element array 20b.

In a case in which a photoacoustic image is generated using four switching patterns illustrated in (I) to (IV) of Fig. 10, and the tip portion of the puncture needle 15 is moved from, for example, the right side to the left side of the piezoelectric element array 20b, the switching pattern is changed in the order of (I) to (IV) of Fig. 10.

In the photoacoustic image generation apparatuses 10 according to the first and second embodiments, the switching pattern illustrated in Fig. 5 is set as the initially set switching pattern. However, in the above-mentioned configuration in which one switching pattern is set as the initially set switching pattern, for example, in a case in which the puncture needle 15 is inserted into the vicinity of the boundary between the receiving region I and the receiving region II in the switching pattern illustrated in Fig. 5, there is a possibility that the position of the tip portion of the puncture needle 15 will not be appropriately detected.

For this reason, before the position of the tip portion of the puncture needle 15 is detected, a plurality of switching patterns may be alternately set as the initial setting and each photoacoustic image corresponding to each switching pattern may be alternately generated such that the tip position detection unit 29 can detect the position of the tip portion of the puncture needle 15 on the basis of any one of the photoacoustic images. Specifically, for example, the switching pattern illustrated in Fig. 5 and the switching pattern illustrated in Fig. 7 may be set as the initial settings and each photoacoustic image corresponding to each switching pattern may be alternately generated.

The invention is not limited to the configuration in which the switching patterns are set before the position of the tip portion of the puncture needle 15 is detected. For example, even in a case in which the tip position detection unit 29 is not capable of detecting the position of the tip portion of the puncture needle 15 due to, for example, some causes after the puncture needle 15 is inserted into the subject, the control unit 28 may alternately set a plurality of switching patterns and alternately generate each photoacoustic image corresponding to each switching pattern such that the tip position detection unit 29 can detect the position of the tip portion of the puncture needle 15 on the basis of any one of the photoacoustic images.

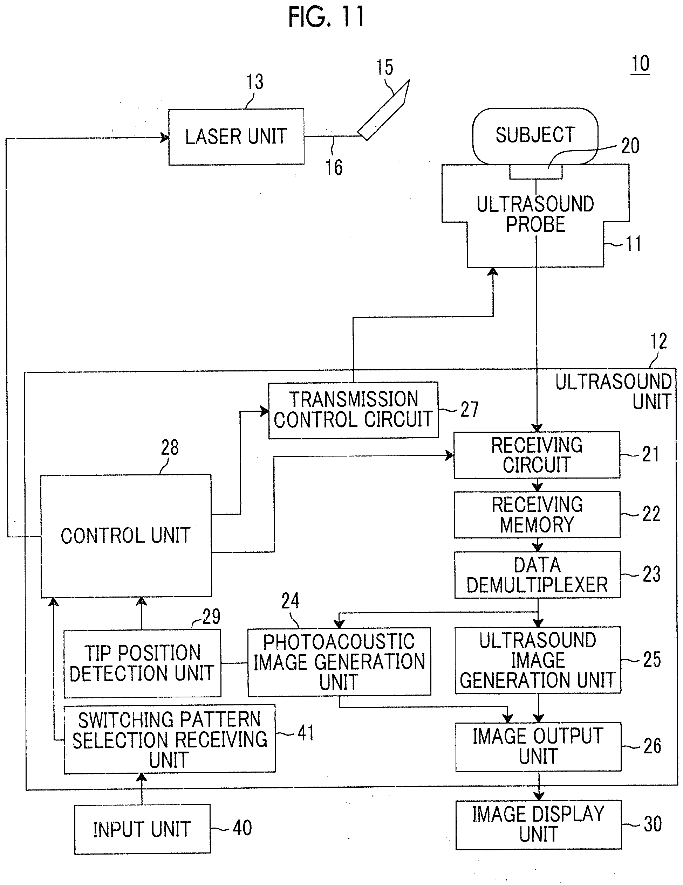

In addition, the user may select the initially set switching pattern before the position of the tip portion of the puncture needle 15 is detected. Specifically, as illustrated in Fig. 11, a switching pattern selection receiving unit 41 that receives the selection of the initially set switching pattern may be provided and the control unit 28 may set the switching pattern received by the switching pattern selection receiving unit 41 as the initial setting. For the selection of the initially set switching pattern, for example, an icon indicating the switching pattern illustrated in Fig. 5 and an icon indicating the switching pattern illustrated in Fig. 7 may be displayed on the image display unit 30 such that the user can select any one of the icons.

In addition, for the selection of the initially set switching pattern, the selection of the switching pattern may not be directly received unlike the above, but the selection of the switching pattern may be received by, for example, receiving information indicating whether the selection of a highlighting process for the puncture needle 15 is present or absent. Here, the highlighting process for the puncture needle 15 is a process of highlighting the image of the puncture needle 15 in an ultrasound image without using a photoacoustic image and is, for example, a contrast enhancement process. In a case in which the user performs a needling operation with the puncture needle 15, a technique, such as a so-called parallel method or a so-called crossing method, is used. The parallel method is a method which performs needling such that the piezoelectric element array 20b of the ultrasound probe 11 is disposed along the length direction of the puncture needle 15. The crossing method is a method which performs needling such that the piezoelectric element array 20b of the ultrasound probe 11 is disposed along a direction intersecting the length direction of the puncture needle 15. The image of the puncture needle 15 appears in a linear shape in a photoacoustic image acquired by the parallel method. The image of the puncture needle 15 appears in a dot shape in a photoacoustic image acquired by the crossing method.

The highlighting process highlights a linear image acquired by the parallel method. Therefore, in a case in which the user selects the highlighting process for the puncture needle 15, it can be determined that a photoacoustic image has been acquired by the parallel method. In the parallel method, the puncture needle 15 is inserted in one of the left and right directions with respect to the piezoelectric element array 20b of the ultrasound probe 11. Therefore, it is preferable that the initially set switching pattern is the switching pattern illustrated in Fig. 5.

In contrast, in a case in which the user does not select the highlighting process for the puncture needle 15, it can be determined that a photoacoustic image has been acquired by the crossing method. In the crossing method, the puncture needle 15 is inserted from the vicinity of the center of the piezoelectric element array 20b of the ultrasound probe 11. Therefore, it is preferable that the initially set switching pattern is the switching pattern illustrated in Fig. 7.

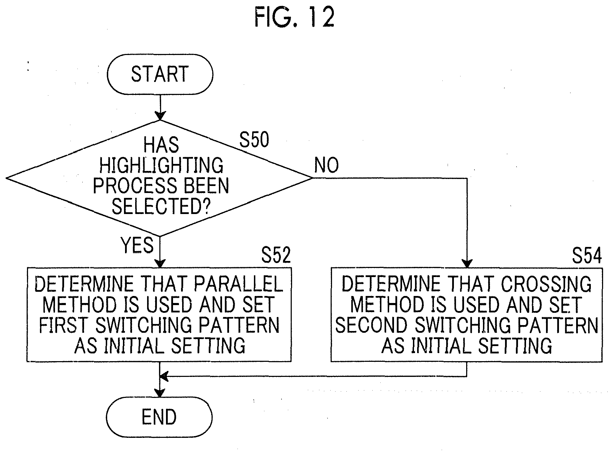

Therefore, from the above-mentioned point of view, it is preferable to select the initially set switching pattern on the basis of a flowchart illustrated in Fig. 12. That is, the control unit 28 checks whether the user has selected the highlighting process for the puncture needle 15. In a case in which the highlighting process has been selected (S50, YES), the control unit 28 determines that a photoacoustic image is acquired by the parallel method and sets the first switching pattern illustrated in Fig. 5 as the initial setting (S52). On the other hand, in a case in which the highlighting process has not been selected (S50, NO), the control unit 28 determines that a photoacoustic image is acquired by the crossing method and sets the second switching pattern illustrated in Fig. 7 as the initial setting (S54).

In the above-described embodiment, the puncture needle 15 is used as an embodiment of the insert. However, the invention is not limited thereto. The insert may be a radio-frequency ablation needle including an electrode that is used for radio-frequency ablation, a catheter that is inserted into a blood vessel, or a guide wire for a catheter that is inserted into a blood vessel. Alternatively, the insert may be an optical fiber for laser treatment.

The needle according to the invention is not limited to a needle, such as an injection needle, and may be a biopsy needle used for biopsy. That is, the needle may be a biopsy needle that is inserted into an inspection target of the living body and extracts the tissues of a biopsy site of the inspection target. In this case, photoacoustic waves may be generated from an extraction portion (intake port) for sucking and extracting the tissues of the biopsy site. In addition, the needle may be used as a guiding needle that is used for insertion into a deep part, such as a part under the skin or an organ inside the abdomen.

The invention has been described above on the basis of the preferred embodiments. However, the insert and the photoacoustic measurement device according to the invention are not limited only to the above-described embodiments. Various modifications and changes of the configurations according to the above-described embodiments are also included in the scope of the invention.

### Explanation of References

10: photoacoustic image generation apparatus
11: ultrasound probe
12: ultrasound unit
13: laser unit
15: puncture needle
15a: puncture needle main body
15b: optical fiber
15c: photoacoustic wave generation portion
15d: hollow portion
16: optical cable
20: acoustic wave detection unit
20a: piezoelectric element
20b: piezoelectric element array
20c: multiplexer
21: receiving circuit
22: receiving memory
23: data demultiplexing unit
24: photoacoustic image generation unit
25: ultrasound image generation unit
26: image output unit
27: transmission control circuit
28: control unit
29: tip position detection unit
30: image display unit
40: input unit
41: pattern selection receiving unit
70: optical cable
C: boundary between receiving regions
P: tip portion of puncture needle

## Claims

1. A photoacoustic image generation apparatus comprising:
an insert of which at least a tip portion is inserted into a subject and which includes a light guide member that guides light to the tip portion and a photoacoustic wave generation portion that absorbs the light guided by the light guide member and generates photoacoustic waves;
an acoustic wave detection unit having a detection element array in which a plurality of detection elements that detect the photoacoustic waves are arranged;
a control unit that controls the acoustic wave detection unit to specify some detection element groups in the detection element array and switches the detection element groups to switch receiving regions of the photoacoustic waves, thereby acquiring detection signals of all of the receiving regions in the detection element array;
a photoacoustic image generation unit that generates a photoacoustic image on the basis of the detection signals of the photoacoustic waves; and
a tip position detection unit that detects a position of the tip portion of the insert on the basis of the photoacoustic image,
wherein the control unit specifies the detection element groups on the basis of the position of the tip portion of the insert detected by the tip position detection unit.

2. The photoacoustic image generation apparatus according to claim 1,
wherein the tip position detection unit detects the position of the tip portion of the insert in time series on the basis of the photoacoustic image generated in time series, and
the control unit sequentially specifies the detection element groups on the basis of the position of the tip portion of the insert detected in time series.

3. The photoacoustic image generation apparatus according to claim 1,
wherein the control unit stores a plurality of switching patterns for the detection element groups in advance, and
the control unit selects any one of the plurality of switching patterns on the basis of the position of the tip portion of the insert and switches the detection element groups using the selected switching pattern.

4. The photoacoustic image generation apparatus according to claim 3,
wherein, in a case in which the detection element array is formed by arranging the plurality of detection elements in at least one row and the arrangement direction is a left-right direction, one of the switching patterns for the detection element groups is a pattern of switching two detection element groups, that is, a detection element group in a left half of the detection element array and a detection element group in a right half of the detection element array.

5. The photoacoustic image generation apparatus according to claim 4,
wherein one of the switching patterns for the detection element groups is a pattern of switching two detection element groups, that is, a detection element group in a central portion of the detection element array and a detection element group on the left and right sides of the central portion of the detection element array, and
the control unit selects one of the pattern of switching the two detection element groups, that is, the detection element group in the left half of the detection element array and the detection element group in the right half of the detection element array and the pattern of switching the two detection element groups, that is, the detection element group in the central portion of the detection element array and the detection element group on the left and right sides of the central portion of the detection element array, on the basis of the position of the tip portion of the insert.

6. The photoacoustic image generation apparatus according to claim 5,
wherein a boundary between a receiving region of the detection element group in the left half of the detection element array and a receiving region of the detection element group in the right half of the detection element array is included in a receiving region of the detection element group in the central portion of the detection element array.

7. The photoacoustic image generation apparatus according to any one of claims 3 to 6,
wherein, in a case in which a distance between the position of the tip portion of the insert and the boundary between the receiving regions of the detection element groups is equal to or less than a predetermined threshold value, the control unit changes the switching pattern.

8. The photoacoustic image generation apparatus according to any one of claims 3 to 7,
wherein the control unit alternately sets the plurality of switching patterns before the position of the tip portion of the insert is detected, and
the tip position detection unit detects the position of the tip portion of the insert on the basis of each photoacoustic image corresponding to each of the switching patterns.

9. The photoacoustic image generation apparatus according to any one of claims 3 to 8,
wherein, in a case in which the tip position detection unit is not capable of detecting the position of the tip portion of the insert, the control unit alternately sets the plurality of switching patterns, and
the tip position detection unit detects the position of the tip portion of the insert on the basis of each photoacoustic image corresponding to each of the switching patterns.

10. The photoacoustic image generation apparatus according to any one of claims 3 to 7 and claim 9, further comprising:
a switching pattern selection receiving unit that receives a selection of the switching pattern initially set before the position of the tip portion of the insert is detected.

11. The photoacoustic image generation apparatus according to claim 10,
wherein the switching pattern selection receiving unit receives a selection of a highlighting process for the insert as the selection of the initially set switching pattern.

12. The photoacoustic image generation apparatus according to any one of claims 1 to 11,
wherein the insert is a needle that is inserted into the subject.
